# EUROPEAN PATENT SPECIFICATION

(11) **EP 1 539 034 B1**
(45) Date of publication and mention of the grant of the patent: **30.09.2015**
(21) Application number: 02761224.1
(22) Date of filing: 01.08.2002
(51) Int. Cl.: A61F 2/04, A61B 17/12

(54) **A DEVICE FOR IMPROVING CARDIAC FUNCTION**
VORRICHTUNG ZUR VERBESSERUNG DER HERZFUNKTION
DISPOSITIF DESTINE A AMELIORER LA FONCTION CARDIAQUE

(43) Date of publication of application: 15.06.2005
(60) Divisional application: 11007994.4
(73) Proprietor: Cardiokinetix, Inc., Menlo Park, CA 94025 (US)
(72) Inventor: SHARKEY, Hugh, R., Redwood City, CA 94062 (US); NIKOLIC, Serjan, D., San Francisco, CA 94121 (US); RADOVANCEVIC, Branislav, Houston, Texas 77030 (US)
(74) Representative: Potter Clarkson LLP
(86) International application number: PCT/US2002/024620
(87) International publication number: WO 2004/012629

(56) References cited:
- WO-A-00/27292
- WO-A-01/30266
- US-A- 3 874 388
- US-A- 5 634 942
- US-A- 6 036 720
- US-A- 6 093 199

## Description

### BACKGROUND OF THE INVENTION

### 1). Field of the Invention

This invention relates to a method and device for improving cardiac function.

### 2). Discussion of Related Art

Congestive heart failure annually leads to millions of hospital visits internationally. Congestive heart failure is a description given to a myriad of symptoms that can be the result of the heart's inability to meet the body's demand for blood flow. In certain pathological conditions, the ventricles of the heart become ineffective in pumping the blood, causing a back-up of pressure in the vascular system behind the ventricle.

The reduced effectiveness of the heart is usually due to an enlargement of the heart. A myocardial ischaemia may, for example, cause a portion of a myocardium of the heart to lose its ability to contract. Prolonged ischemia can lead to infarction of a portion of the myocardium (heart muscle) wherein the heart muscle dies and becomes scar tissue. Once this tissue dies it no longer functions as a muscle and cannot contribute to the pumping action of the heart. When the heart tissue is no longer pumping effectively, that portion of the myocardium is said to be hypokinetic, meaning that it is less contractile than the uncompromised myocardial tissue. As this situation worsens, the local area of compromised myocardium may in fact bulge out as the heart contracts, further decreasing the heart's ability to move blood forward. When local wall motion moves in this way it is said to be dyskinetic. The dyskinetic portion of the myocardium may stretch and eventually form an aneurysmic bulge. Certain diseases may cause a global dilated myopathy, i.e., a general enlargement of the heart when this situation continues for an extended period of time. As the heart begins to fail, the filling pressures increase, which stretches the ventricular chamber prior to contraction, greatly increasing the pressure (preload) to the heart. In response, the heart tissue remodels to accommodate the chronically increased filling pressures, further increasing the work that the now-compromised myocardium must perform. This vicious cycle of cardiac failure results in the symptoms of congestive heart failure such as shortness of breath on exertion, edema in the periphery, nocturnal dypsnia (a characteristic shortness of breath that occurs at night after going to bed), weight gain, and fatigue, to name a few. The enlargements increase stress on the myocardium. The stress increase requires a larger amount of oxygen supply, which can result in exhaustion of the myocardium leading to a reduced cardiac output of the heart.

WO 01/030266 discloses a filter device that is placed across the ostium of a left atrial appendage. The device includes a radially expandable support structure to which is attached a filtering membrane that is configured to extend across the ostium of the left atrial appendage. The filtering membrane is permeable and allows blood to pass but prevents passage of a thrombus.

WO 00/27292 discloses a device for occluding a left atrial appendage. The device comprises an occluding member and a stabilizing member, both of which are enlargeable from a reduced cross-section to an enlarged cross-section. The occlusion member comprises an expandable frame and a membrane.

### SUMMARY OF THE INVENTION

This invention relates to a device for improving cardiac function according to claim 1. The device has a frame construction that is movable from a collapsed state, wherein the frame construction has a small cross-dimension to allow the frame construction to be fed through a tubular passage with a small diameter into the heart, to an expanded state wherein the frame construction, after leaving the tubular passage and having been located in an installed position in a ventricular cavity of the heart, has a cross-dimension substantially larger than the small diameter of the tubular passage and approximating a cross-dimension of ventricular cavity where the frame construction is positioned. The device has at least one anchor formation connected to the frame construction, having at least one anchoring portion that is positioned and capable of anchoring to tissue of a myocardium of the heart, and so anchor the frame construction in the installed position to the myocardium. The device further has a membrane which is in a folded condition when being fed through the tubular passage, and in an unfolded condition after leaving the tubular passage, in the unfolded condition having an area substantially larger than a cross-sectional area of the tubular passage, and being secured to the frame construction in a position to substantially form a division between volumes of the ventricular cavity on opposing sides of the membrane.

According to one aspect of the invention, at least two anchoring formations are connected to the frame construction, and are spaced from one another to allow for positioning of the frame construction at a select angle relative to the ventricular cavity.

According to another aspect of the invention, the frame construction is deformable into various non-circular shapes to allow for positioning thereof in ventricular cavities having differing non-circular shapes.

According to a further aspect of the invention, the frame construction includes a support frame next to and supporting the membrane, the support frame being sufficiently strong to support the membrane when a ventricular pressure acts on the membrane.

### BRIEF DESCRIPTION OF THE DRAWINGS

The invention is further described by way of examples with reference to the accompanying drawings, wherein:
Figure 1A is a perspective view of a main frame of a device, according to an embodiment of the invention, for improving cardiac function;
Figure 1B is a view similar to Figure 1A, illustrating the main frame in hidden lines and further illustrating in solid lines a support frame of the device mounted to the main frame;
Figure 1C is a top plan view illustrating a membrane of the device secured on top of the support frame;
Figure 2A is a cross-sectional side view of a heart, a catheter that is inserted into a left ventricle of the heart, and the device as it is packaged within an end of the catheter;
Figure 2B is a perspective view illustrating a device manipulating apparatus within the end of the catheter;
Figures 3A-3D illustrate how the device is secured to a myocardium of the heart;
Figures 4A-4B are graphs illustrating the pressures within the left atrium and the left ventricle, respectively;
Figures 5A-5C illustrate how the device can be mounted with the support frame to support the membrane in a different plane;
Figure 6 is a top plan view illustrating a larger device, according to another embodiment of the invention, mounted in a lower portion within a left ventricle of a heart;
Figures 7A-7B are perspective views from different sides, illustrating components of a device according to a further embodiment of the invention;
Figure 8 is a cross-sectional side view illustrating a sheet that is curved to substantially conform to an inner wall of a heart;
Figure 9 is a cross-sectional end view on 9-9 in Figure 8;
Figure 10 is a cross-sectional side view illustrating a device that is used for closing off a small ventricle of a heart; and
Figure 11 is a cross-sectional side view illustrating the same device as in Figure 10, used for closing off a large ventricle of a heart.

### DETAILED DESCRIPTION OF THE INVENTION

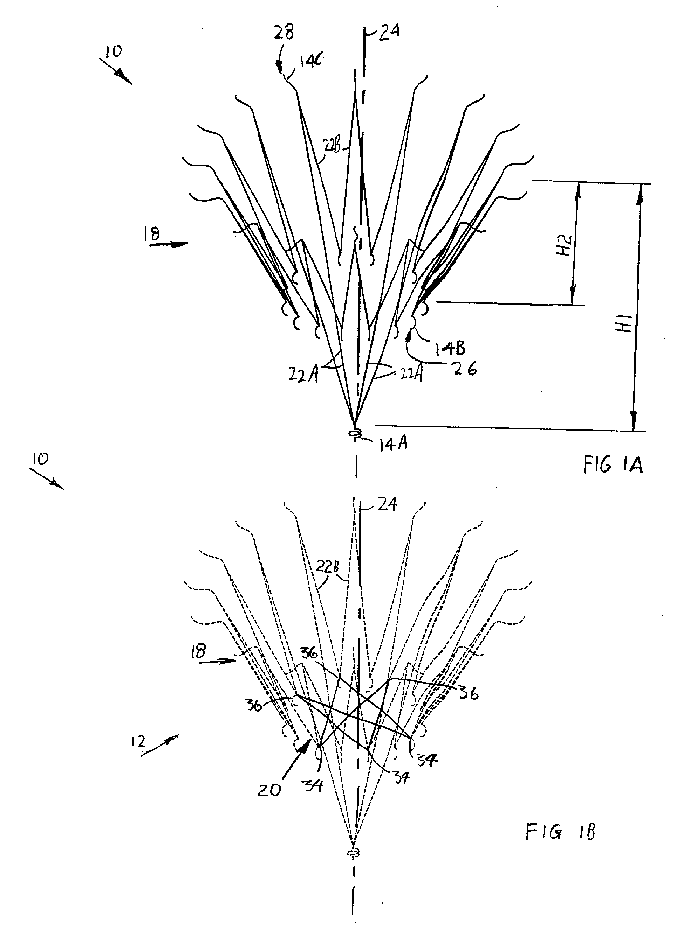

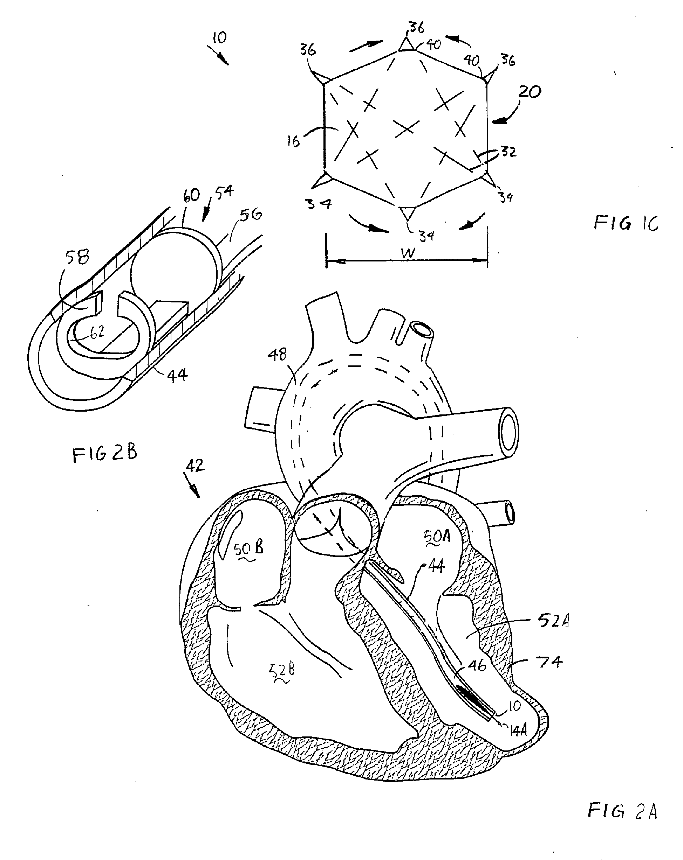

Figures 1A, 1B, and 1C illustrate components of a device 10, according to an embodiment of the invention, for improving cardiac function. The device 10 includes a frame construction 12, a plurality of anchoring formations 14, and a membrane 16. The frame construction 12 includes a main frame 18 and a support frame 20 secured to the main frame 18. The membrane 16 is secured on top of the support frame 20.

As shown in Figure 1A, the main frame 18 includes a sequence or series of segments 22. Even segments of the series extend in an upward direction, and odd segments extend downward. The sequence formed by the segments 22 entirely surrounds a vertical axis 24. Movement of the segments 22 toward one another causes collapse of the main frame 18 toward the vertical axis 24. The frame construction 12 is made of a biocompatible wire-like shape-memory material, for example, nickel-titanium.

The anchoring formations 14 include a distal anchoring screw 14A, distal anchoring hooks 14B, and proximal anchoring hooks 14C. Two or more (in the present example, four) of the segments 22A are longer, and extend further down than other ones of the segments 22B. The segments 22A have their lower ends connected to one another, and the distal anchoring screw 14A is secured to the lower ends of the segments 22A. The segments 22A and 22B may be curved, as opposed to being straight as shown in the figures.

The distal anchoring hooks 14B are secured to lower ends of the segments 22B. Each distal anchoring hook 14B curves out and then down and is formed with a lower sharp end 26.

The proximal anchoring hooks 14C are secured to upper ends of the segments 22A and 22B. Each one of the proximal anchoring hooks 14C curves out and then up and terminates in an upper sharp end 28. The anchoring hooks 14B and 14C move together with the main frame 18 toward the vertical axis 24 when the main frame 18 is collapsed.

As shown in Figure 1C, the support frame 20 includes six (or more) elements 32, sequentially after one another and overlaying one another to form a six-pointed star. The elements 32 can pivot in a scissor-like manner relative to one another. Pivoting of the elements 32 relative to one another moves corners 34 of the star toward one another, while corners 36 on an opposing side of the star move toward one another. The support frame 20 then has an elongated configuration with the corners 36 at one end and the corners 34 at an opposing end.

Referring to Figure 1B, each corner 36 is positioned around and slidably secured to a respective one of the segments 22B. When the main frame 18 is collapsed, the corners 34 slide up the segments 22B to which they are secured, while the corners 36 remain at the bottom of the segments 22B to which they are secured. When the main frame 18 is fully collapsed, the support frame 20 is in the form of an elongated arrangement extending along the vertical axis 24, with the corners 34 at the top and the corners 36 at the bottom.

Figure 1C also shows the membrane 16, in an unfolded condition, secured on the elements 32 of the support frame 20. An edge 40 of the membrane 16 is secured to the elements 32. Two of the elements 32 form a cross below a center of the membrane 16, and the other four elements 32 support the membrane 16 between the cross and the edge 40. Collapse of the support frame 20 folds the membrane 16 into an elongated folded arrangement extending along the elongated arrangement formed by the collapsed support frame 20. The membrane 16 is made of a biocompatible foldable material, for example Gore-Tex^{®}, poly-ethylene terephthalate, or polypropylene mesh.

Figure 2A illustrates the device 10 that is inserted into a heart 42 by means of a catheter 44. The device 10 is collapsed and is inserted into an end of the catheter 44. The axis 24, shown vertically in Figures 1A and 1B, now extends along an axis of an elongated tubular passage 46 in the catheter 44. The device 10 is packaged with the distal anchoring screw 14A protruding from the end of the catheter 44. The catheter 44 is non-invasively steered through the aorta 48 and the aortic valve (not shown) into the left ventricle 52A of the heart 42. The other chambers of the heart 42 are the right ventricle 52B, the left atrium 50A, and the right atrium 50B.

As shown in Figure 2B, a device manipulating apparatus 54 is disposed within the catheter 44. The apparatus 54 includes an elongated manipulator 56, a rotator piece 58, and a support piece 60. Only a distal portion of the elongated manipulator 56 is shown. A handle (not shown) is attached to a proximal portion of the elongated manipulator 56. The elongated manipulator 56 can bend to conform to the curved or bent shape of the catheter 44, but is relatively rigid against a torque about an elongated axis thereof. The rotator piece 58 is secured to an end of the elongated manipulator 56, and the support piece 60 is secured to the elongated manipulator 56 slightly proximal to the rotator piece 58. The rotator piece 58 has an internal device engaging formation 62. The device 10 is inserted into the formation 62 until proximal surfaces of the device 10 contact the support piece 60. The formation 62 conforms to an outer shape of the device 10, so that the device 10 rotates together with the rotator piece 58 when the rotator piece 58 is rotated by the elongated manipulator 56. The device 10 may be fed out of an end of the catheter 44 by the support piece 60 when the elongated manipulator 56 is advanced in an elongated direction of the catheter 44. The support piece 60 also prevents movement of the device 10 in an opposite direction into the catheter 44.

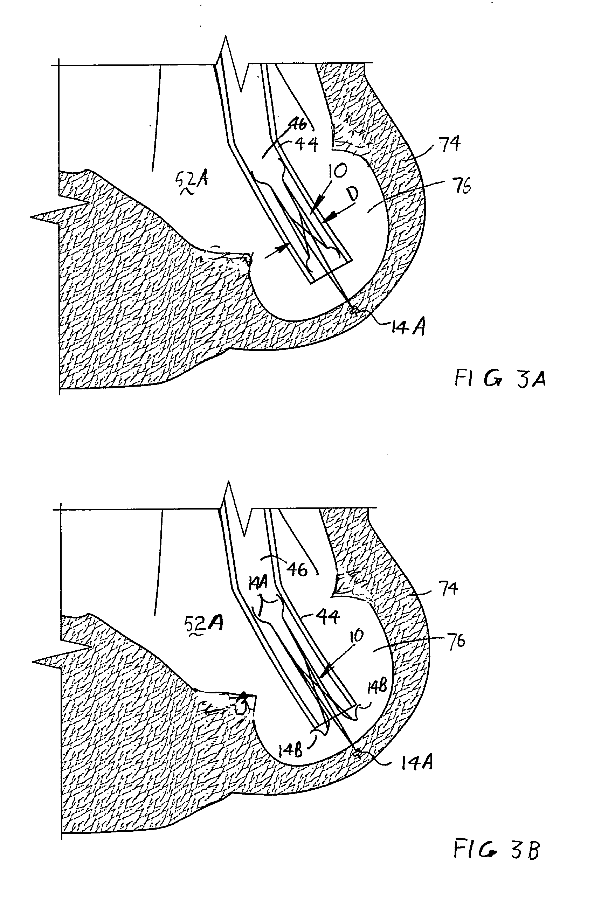

Reference is now made to Figure 3A. The myocardium 74 of the heart has formed an aneurysmic formation or bulge 76 out of the left ventricle 52A. A previous infarction, or cessation of blood supply, to the portion of the myocardium 74 now forming the bulge 76. Continuous exposure of the dyskinetic portion of the myocardium 74 to high pressures in the left ventricle 52A has caused the aneurysmic bulge 76.

The catheter 44 is steered so that the distal anchoring screw 14A contacts a base of the bulge 76. The catheter 44 is then rotated so that the distal anchoring screw 14A screws into the myocardium 74 at a target site the base of the bulge 76.

As shown in Figure 3B, the catheter 44 is then retracted over the device 10, with the distal anchoring screw 14A anchoring the frame construction 12 to the myocardium 74 at the base of the bulge 76. The distal anchoring hooks 14B leave the catheter 44 as the catheter 44 is retracted, before the remainder of the device 10, and bend outwardly under spring action.

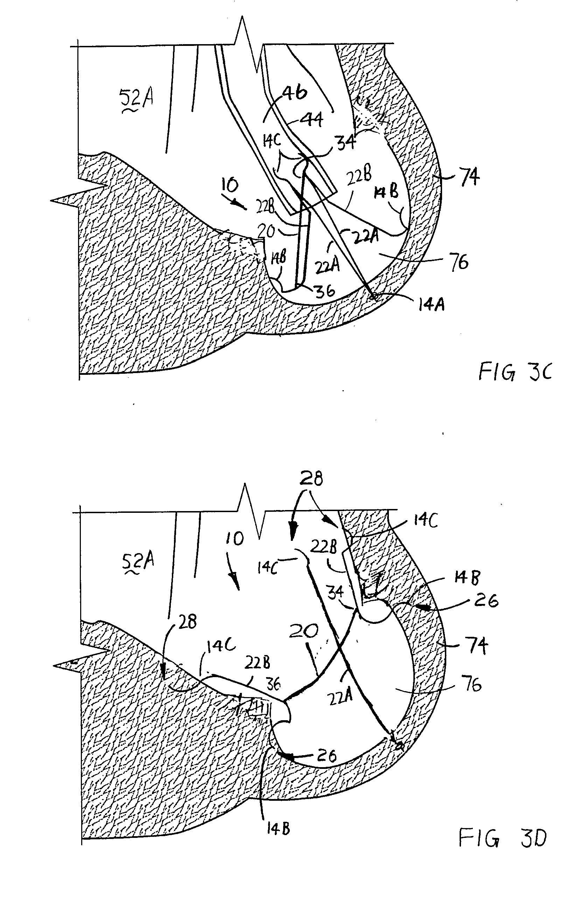

As shown in Figure 3C, further withdrawal of the catheter 44 from the segments 22B causes the segments 22B to spring outwardly, and the distal anchoring hooks 14B to come into contact with the myocardium 74. The support frame 20 pivots away from its alignment with the center axis of the elongated tubular passage 46, and the proximal anchoring hooks 14C are at this stage still located within the elongated tubular passage 46.

As shown in Figure 3D, the catheter 44 is subsequently withdrawn from proximal anchoring hooks 14C. Proximal portions of the segments 22A and 22B spring outwardly after the proximal anchoring hooks 14C leave the tubular passage 46, so that the proximal anchoring hooks 14C move outwardly into contact with the myocardium 74. A proximal portion of each segment 22A or 22B pivots relative to a distal portion thereof. Pivoting of the segments 22B rotates the lower sharp ends 26 of the distal anchoring hooks 14B into the myocardium 74. Embedding of the distal anchoring hooks 14B into the myocardium 74 anchors the segments 22B to the myocardium 74. Beating of the heart 42 causes relative movement between the myocardium 74 and proximal anchoring hooks 14C, so that the upper sharp ends 28 may also penetrate the myocardium 74. The proximal anchoring hooks 14C are thereby also embedded into the myocardium 74, and anchor proximal portions of the segments 22A and 22B to the myocardium 74. Each segment 22A or 22B is near the myocardium 74 at all locations along the length of the respective segment 22A or 22B, and is anchored to the myocardium 74 through the anchoring formations 14.

The corners 34 of the support frame 20 slide along the segments 22B to which they are secured when the segments 22B rotate outwardly relative to one another. When comparing Figure 3D with Figure 3C, it can be seen that the support frame 20 is in a plane which is substantially at right angles with respect to the axis of the elongated tubular passage 46. The membrane 16 (Figure 1C) unfolds and is supported on top of the support frame 20. The membrane 16 forms a division between the aneurysmic bulge 76 and a remainder of the left ventricle 52A.

After the device 10 is installed, the aneurysmic bulge 76, having been segregated from the remainder of the left ventricle 52A, eventually clots off behind the membrane 16, thereby effectively reducing the internal volume in the left ventricle 52A. Stretching of the portion of the myocardium 74 forming the aneurysmic bulge 76 is also effectively eliminated. By blocking off a portion of the left ventricle 52A not contributing to pumping during a systolic portion of a pump cycle, properly functioning portions of the myocardium 74 can contract normally and use up a normal amount of oxygen. By reducing the amount of oxygen uptake during a given period of time, properly functioning portions of the myocardium 74 are not exhausted and can continue to function properly. Cardiac output increases and the likelihood of congestive heart failure is reduced.

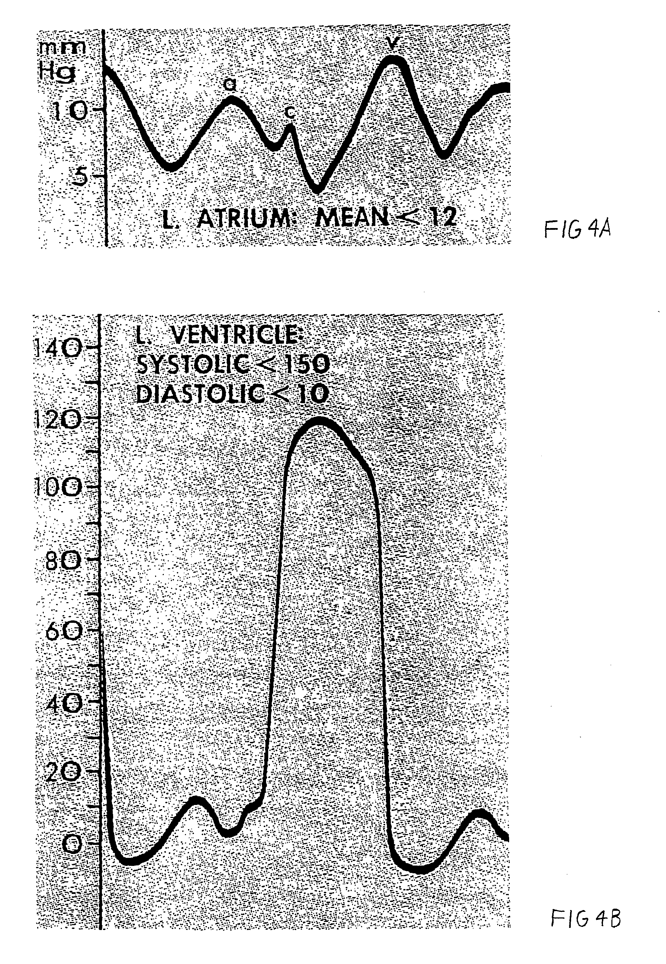

Figures 4A and 4B illustrate pressures within the left atrium 50 and the left ventricle 52A, respectively, of a healthy human being. It can be seen that the peak left ventricular pressure, i.e., the pressure in the left ventricle 52A during the systolic portion, reaches approximately 120 mm Hg. This pressure acts directly on the membrane 16. It can be assumed that the pressure on an opposing side of the membrane 16, i.e., the side of the aneurysmic bulge 76, is close to zero. The support frame 20 supports the sheet 16 at a sufficient number of locations and is sufficiently strong to prevent the membrane 16 from collapsing during peak systolic pressure. An peak pressure in the region of 50 to 60 mm Hg for a sustained period of a few hours is generally regarded as being incompatible with life.

In the given example, there are a total of 31 anchoring formations 14, including the distal anchoring screw 14A, 14 distal anchoring hooks 14B, and 16 proximal anchoring hooks 14C. The large number of anchoring formations 14 ensure proper anchoring to the myocardium 74. The large number of anchoring formations 14 also allows for positioning of the membrane 16 at a select location within the left ventricle 52A and at a select angle and within a select plane relative to the myocardium 74. The anchoring formations 14, and in particular the anchoring hooks 14B and 14C, their shape, orientation, and placement, are thus uniquely suited for anchoring of the frame construction 12, especially when compared with other anchoring formations such as pins, clamps, staples, screws, and surgical thread. What should also be noted is that the anchoring formations 14 penetrate through only a portion of the myocardium 74, and thus do not damage the pericardium. What should further be noted is that none of the anchoring formations 14 or other components of the device 10 can bump against the myocardium 74, to avoid electrostimulation of the myocardium 74 that can lead to arrhythmias.

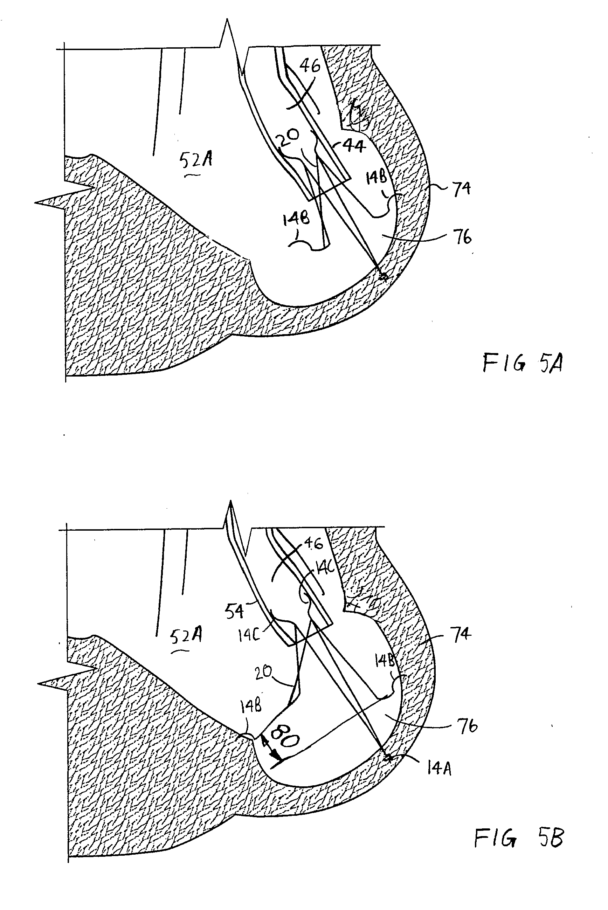

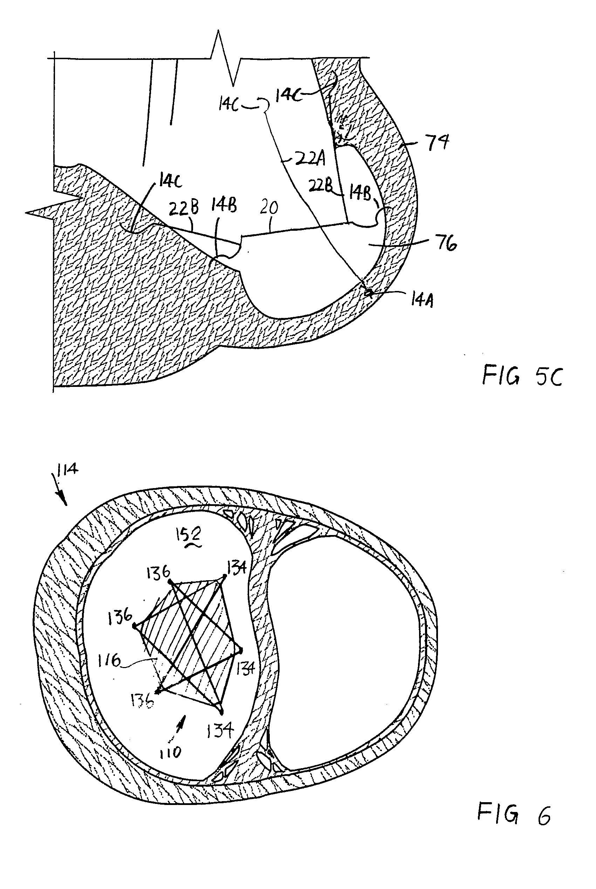

Figures 5A, 5B, and 5C illustrate one manner in which the support frame 20 and the membrane 16 can be positioned at a select angle relative to the myocardium 74. When comparing Figure 5A with Figure 3C, it can be seen that the catheter 44 is positioned closer to a right side (as viewed) of the myocardium 74. The distal anchoring hooks 14B on the right engage with the myocardium 74 before the distal anchoring hooks 14B on the left engage with the myocardium 74. Further withdrawal of the catheter 44, as shown in Figure 5B, results in engagement of the distal anchoring hooks 14B on the left with the myocardium 74 at a location which is displaced by an offset distance 80 in a direction of an axis of the elongated tubular passage 46. When comparing Figure 5C with Figure 5B, it can be seen that, due to the offset distance 80, the support frame 20 is eventually at an angle of approximately 60° relative to the axis of the elongated tubular passage 46. Although not blocking a mouth of the aneurysmic bulge 76, this serves to illustrate that the membrane 16 can be positioned in different select planes, as may be required, due to the flexibility of the frame construction 12 and various virtual triangles that are formed by connecting locations where the anchoring formations 14 anchor to the myocardium 74.

Referring again to Figures 1A, 1C, and 3A, the main frame 18 has a vertical height H1, a height from the distal anchoring hooks 14B to the proximal anchoring hooks 14C H2, the membrane 16 has a width W, and the elongated tubular passage 46 has a diameter D. These dimensions can be modified according to requirement, and the following table lists a number of examples:

| **H1** | **H2** | **W** | **D** |
|---|---|---|---|
| 6 cm | 3 cm | 2.5 cm | 1 cm |
| 7 cm | 4 cm | 3 cm | 1.2 cm |
| 8 cm | 5 cm | 4 cm | 1.5 cm |
| 8.5 cm | 5.5 cm | 5 cm | 2 cm |
| 9.5 cm | 6 cm | 6 cm | 2.2 cm |
| 9.5 cm | 8 cm | 7 cm | 2.6 cm |

The first row in the table lists the dimensions for the device 10 hereinbefore described which is used for blocking a relatively small aneurysmic bulge 76. Larger aneurysmic bulges can be blocked using slightly larger devices. As mentioned, certain diseases may cause general enlargement of endocardial cavities of a heart without necessarily creating a specific identifiable bulge. Larger devices can be used to block portions of these enlarged endocardial cavities. In such cases, it may also be possible to use two devices in a side-by-side arrangement or with their membranes overlapping one another.

Figure 6 illustrates one such a larger device 110 that is inserted in the bottom of the left ventricle 152 of a heart 114. The main frame (not shown) of the device 110 is formed into a non-circular shape, so that an outline formed by corners 134 and 136 of a support frame of the device define a non-circular shape. A membrane 116 mounted on top of the support frame also defines a non-circular shape. The shape of the membrane 116 conforms approximately to a non-circular D-shape of the left ventricle 152 at a height where the membrarte 116 is positioned. The same device 110 can be deformed into various different shapes, according to requirement.

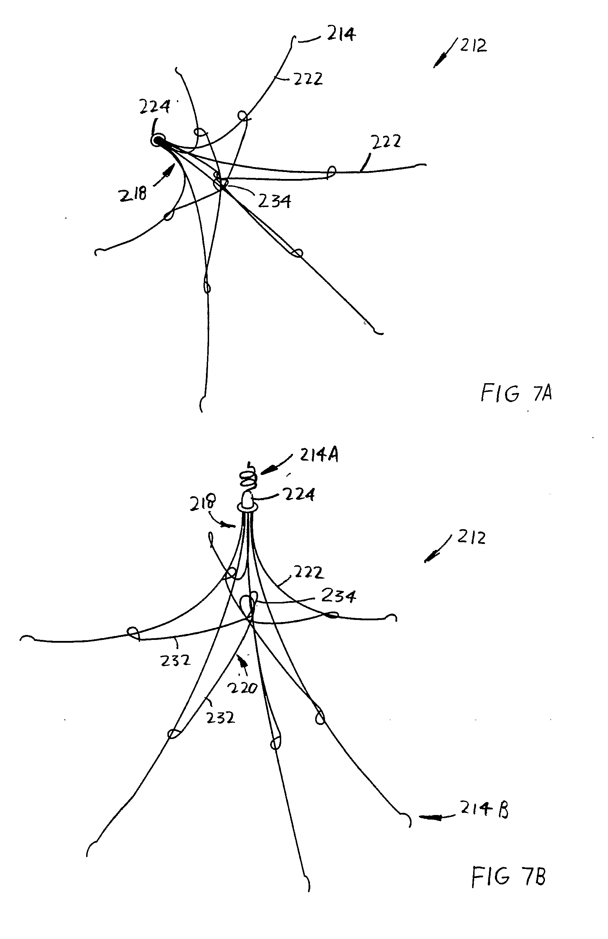

Figures 7A and 7B illustrate a frame construction 212 and anchoring formations 214 of a device according to an alternative embodiment of the invention. The frame construction 212 includes a main frame 218 and a support frame 220. The main frame 218 has a plurality of segments 222 having distal ends connected to one another at a common location 224. Proximal portions of the segments 222 can collapse toward one another and spring outwardly away from one another. The anchoring formations 214 include a distal anchoring screw 214A secured at the common location 224, and proximal anchoring hooks 214B on proximal ends of the segments 222. The support frame 220 includes a plurality of elements 232. The elements 232 have ends that are pivotally connected to one another at a common location 234. An opposing end of each element 232 is slidably secured to a respective one of the segments 222. The manner in which the segments 222 of the main frame 218 collapse is simultaneously replicated by the manner in which the elements 232 of the support frame 220 collapse. In use, the distal anchoring screw 214A is first screwed into a myocardium. A catheter is then withdrawn from the frame construction 212. Once the catheter is entirely removed from the frame construction 212, the proximal anchoring hooks 214B spring outwardly and embed themselves into the myocardium. The support frame 220 simultaneously moves from its collapsed condition into its expanded condition. A membrane (not shown) is secured to, unfolded by, and supported by the support frame 220.

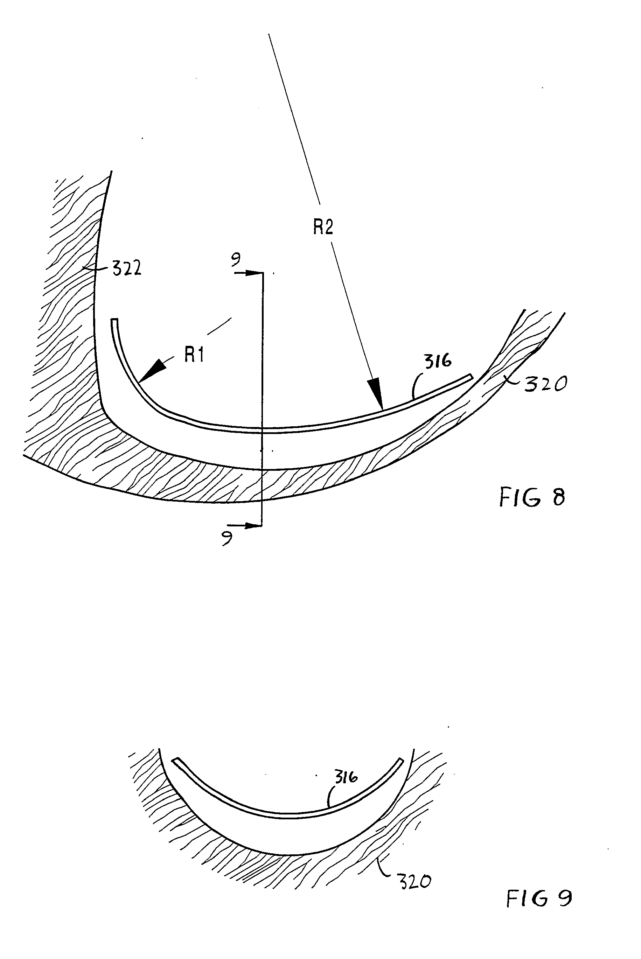

The support frame of a device may be shaped so that a membrane attached to the support frame has a desired shape. Figures 8 and 9, for example, illustrate a membrane 316 that conforms approximately to a shape defined by an anterior wall 320 and a septum 322 of a heart. As shown in Figure 8, the membrane 316 has a portion on the left having a radius R1 and a portion on the right having a radius R2 which is a multiple of the radius R1. The sheet 316 material may be formed to have more than two radii of curvature. Referring to Figure 9, it can be seen that the membrane 316 is curved also when viewed on 9-9 in Figure 8. The curved shape of the membrane 316 allows the membrane 316 to block off larger portions of the anterior wall 320 and the septum 322 without reducing the internal volume of the left ventricle by too great a degree.

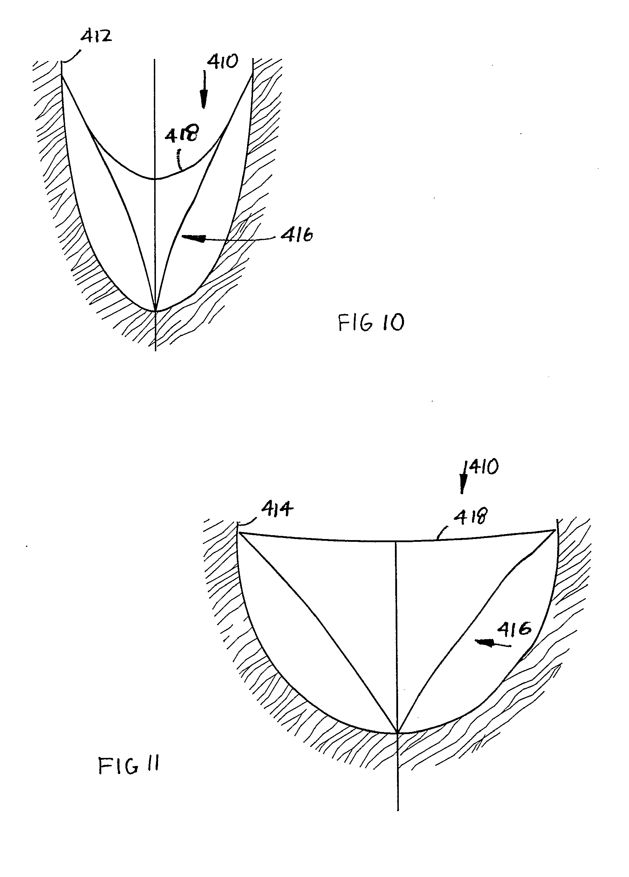

It may also be possible to use the same device to block off either large or small cavities. Figures 10 and 11 illustrate the same device 410 used for closing off a small ventricle 412 and a large ventricle 414, respectively. As in, for example, the embodiment described with reference to Figures 7A and 7B, the device 410 has a frame construction 416 that can spring outwardly, and a membrane 418 secured and expanded by the frame construction 416. The frame construction 416 springs out more in Figure 11 than in Figure 10, and the membrane 416 is accordingly unfolded into a larger cross-sectional shape.

The support frame and anchoring formations of, for example, the device illustrated in Figure 1A may be used for other purposes instead of or in addition to supporting a membrane as described. The frame construction 18 provides an electrically conductive path that can be used for left ventricular pacing. For example, one of the proximal anchoring hooks 14C may engage with and be sufficiently long to penetrate from a left ventricle through a septum into a right ventricle of a heart. A terminal of a pacemaker can then be inserted into the right ventricle and connected to the hook that penetrates through the septum. Electric current can conduct between the terminal of the pacemaker through the main frame 18 to other ones of the anchoring formations 14 connected to the myocardium of the left ventricle. The frame construction 12 also provides a strong support for mounting components that can be used for other purposes, such as an annulus component that can be positioned around the mitral valve, or a component that is used for reshaping a papillary muscle. The device 10 can also be used for delivering of drugs, proteins, stem cells, etc. to the heart.

While certain exemplary embodiments have been described and shown in the accompanying drawings, it is to be understood that such embodiments are merely illustrative and not restrictive of the current invention, and that this invention is not restricted to the specific constructions and arrangements shown and described since modifications may occur to those ordinarily skilled in the art.

## Claims

1. A device (10) for improving cardiac function, comprising:
a frame construction (12) comprising a support frame (20), said frame construction being movable from a collapsed state, to allow the frame construction to be fed through a tubular passage (46) into a heart, to an expanded state wherein the frame construction has a cross-dimension for approximating a cross-dimension of a ventricular cavity;
at least one anchor formation (14) connected to the frame construction, the anchor formation having at least one anchoring portion that is positioned and capable of anchoring to tissue of a myocardium of the heart, and so anchor the frame construction in an installed position to the myocardium; and
a membrane (16) that is moveable from a folded condition, to allow it to be fed through the tubular passage (46) into the heart, to an unfolded condition in which it has an area substantially larger than a cross-sectional area of the tubular passage, said membrane being secured to the frame construction in a position to enable it to substantially form a division between volumes of the ventricular cavity on opposing sides of the membrane when the frame construction is in the installed position,
wherein the support frame is sufficiently strong to support the membrane and prevent it from collapsing during peak systolic pressures of at least 60 mmHg.

2. The device of claim 1, comprising at least two anchor formations (14) connected to the frame construction (12), each anchor formation having at least one anchoring portion that is positioned and capable of anchoring to tissue of a myocardium of the heart, and so anchor the frame construction in the installed position to the myocardium, the anchoring portions being spaced from one another to allow for positioning of the frame construction at a select angle relative to the ventricular cavity.

3. The device of claim 2, comprising a third anchor formation (14), anchor portions of the anchor formations being positioned at corners of a triangle to allow for positioning of the frame construction (12) with the membrane (16) in a select plane relative to the ventricular cavity.

4. The device of claim 2 or 3, wherein the anchoring portions have sharp ends (26, 28) for penetrating the myocardium.

5. The device of claim 4, wherein at least some of the anchoring formations are anchoring hooks (14B, 14C).

6. The device of claim 5, wherein at least some of the anchoring hooks (14B, 14C) are distal anchoring hooks (14B) on a distal portion of the frame construction (12) that rotate into the myocardium while a catheter (44) forming the tubular passage (46) is withdrawn off the frame construction.

7. The device of claim 6, wherein partial withdrawal of the catheter (44) off a distal portion of the frame construction (12) causes expansion of the distal portion of the frame construction and movement of the distal anchoring hooks (14B) away from a center line of the tubular passage (46), so that the sharp ends (26) of the distal anchoring hooks move into contact with the myocardium, and further withdrawal of the catheter off the frame construction causes rotation of the distal anchoring hooks into the myocardium.

8. The device of claim 7, wherein a proximal portion of the frame construction (12) expands after leaving the tubular passage (46), expansion of the proximal portion of the frame construction causing rotation of the distal anchoring hooks (14B) into the myocardium.

9. The device of claim 5, wherein at least some of the anchoring hooks (14B, 14C) are proximal anchoring hooks (14C) on a proximal portion of the frame construction (12), the proximal portion of the frame construction expanding after leaving the tubular passage (46), expansion of the proximal portion of the frame construction causing movement of the proximal anchoring hooks away from a center line of the tubular passage so that the proximal anchoring hooks move into contact with the myocardium.

10. The device of any one of the preceding claims, wherein the frame construction (12) includes a main frame (18) surrounding a vertical axis (24) and comprising a sequence of sections (22, 22A, 22B) that alternate in upward and downward directions, expansion of the frame construction moving the sections apart.

11. The device of claim 10, wherein, when viewed from above, the frame construction (12) is deformable into various non-circular shapes to allow for positioning thereof in ventricular cavities having differing non-circular shapes.

12. The device of any one of claims 1 to 9, wherein, when viewed from above, the frame construction (12) surrounds a vertical axis (24) and is deformable into various non-circular shapes to allow for positioning thereof in ventricular cavities having differing non-circular shapes.

13. The device of any one of the preceding claims, wherein the frame construction (12) further includes a main frame (18) that is configured to expand away from a center line of the tubular passage, and wherein when the main frame is in the expanded state, the support frame (20) is mounted to the main frame within an opening defined by the main frame.

14. The device of claim 13, wherein the support frame (20), when the main frame (18) is in the collapsed state, is collapsed into an elongated arrangement.

15. The device of claim 14, wherein the support frame has at least two elements (32) that pivot relative to one another in a scissor-like manner.

16. The device of any one of the preceding claims, wherein the membrane (16) is within the frame construction (12) when the frame construction is in the collapsed state.

17. The device of any one of the preceding claims, wherein the membrane (16) is at least 3 cm in diameter.

## Patentansprüche

1. Vorrichtung (10) zur Verbesserung der Herzfunktion, Folgendes umfassend:
eine Rahmenkonstruktion (12), die einen Stützrahmen (20) umfasst, wobei die Rahmenkonstruktion aus einem zusammengeklappten Zustand, der ermöglicht, dass die Rahmenkonstruktion durch einen röhrenförmigen Durchlass (46) in ein Herz eingeführt wird, und einem aufgeklappten Zustand, in dem die Rahmenkonstruktion eine Querabmessung aufweist, die einer Querabmessung eines Herzkammerhohlraumes nahekommt, beweglich ist,
mindestens ein Verankerungsgebilde (14), das mit der Rahmenkonstruktion verbunden ist, wobei das Verankerungsgebilde mindestens einen Verankerungsabschnitt aufweist, der zum Verankern an Gewebe eines Herzmuskels positioniert und geeignet ist und damit die Rahmenkonstruktion in einer eingesetzten Position am Herzmuskel verankert, und
eine Membran (16), die aus einem gefalteten Zustand, der ermöglicht, dass sie durch einen röhrenförmigen Durchlass (46) in das Herz eingeführt wird, und einem entfalteten Zustand, in dem sie eine Fläche aufweist, die wesentlich größer als eine Querschnittsfläche des röhrenförmigen Durchlasses ist, beweglich ist, wobei die Membran in einer Position an der Rahmenkonstruktion befestigt ist, die sie in die Lage versetzt, im Wesentlichen eine Trennwand zwischen Volumen des Herzkammerhohlraumes an gegenüberliegenden Seiten der Membran zu bilden, wenn sich die Rahmenkonstruktion in der eingesetzten Position befindet,
wobei der Stützrahmen ausreichend stark ist, die Membran zu stützen und zu verhindern, dass sie bei einem systolischen Höchstblutdruck von mindestens 60 mmHg zusammenbricht.

2. Vorrichtung nach Anspruch 1, mindestens zwei Verankerungsgebilde (14) umfassend, die mit der Rahmenkonstruktion (12) verbunden sind, wobei jedes Verankerungsgebilde mindestens einen Verankerungsabschnitt aufweist, der zum Verankern an Gewebe eines Herzmuskels positioniert und geeignet ist und damit die Rahmenkonstruktion in der eingesetzten Position am Herzmuskel verankert, wobei die Verankerungsabschnitte voneinander beabstandet sind, um ein Positionieren der Rahmenkonstruktion in einem ausgewählten Winkel im Verhältnis zum Herzkammerhohlraum zu positionieren.

3. Vorrichtung nach Anspruch 2, ein drittes Verankerungsgebilde (14) umfassend, wobei Verankerungsabschnitte des Verankerungsgebildes an Ecken eines Dreiecks positioniert sind, um das Positionieren der Rahmenkonstruktion (12) mit der Membran (16) in einer gewählten Ebene im Verhältnis zum Herzkammerhohlraum zu ermöglichen.

4. Vorrichtung nach Anspruch 2 oder 3, wobei die Verankerungsabschnitte scharfe Enden (26, 28) zum Eindringen in den Herzmuskel aufweisen.

5. Vorrichtung nach Anspruch 4, wobei mindestens einige der Verankerungsgebilde Verankerungshaken (14B, 14C) sind.

6. Vorrichtung nach Anspruch 5, wobei mindestens einige der Verankerungshaken (14B, 14C) distale Verankerungshaken (14B) an einem distalen Abschnitt der Rahmenkonstruktion (12) sind, die sich in den Herzmuskel drehen, wenn ein Katheter (44), der den röhrenförmigen Durchlass (46) bildet, von der Rahmenkonstruktion abgezogen wird.

7. Vorrichtung nach Anspruch 6, wobei das teilweise Abziehen des Katheters (44) von einem distalen Abschnitt der Rahmenkonstruktion (12) das Ausdehnen des distalen Abschnitts der Rahmenkonstruktion und die Bewegung der distalen Verankerungshaken (14B) weg von einer Mittellinie des röhrenförmigen Durchlasses (46) bewirkt, so dass sich die scharfen Enden (26) der distalen Verankerungshaken in den Kontakt mit dem Herzmuskel bewegen, und das weitere Abziehen des Katheters von der Rahmenkonstruktion die Drehung der distalen Verankerungshaken in den Herzmuskel bewirkt.

8. Vorrichtung nach Anspruch 7, wobei sich ein proximaler Abschnitt der Rahmenkonstruktion (12) nach dem Verlassen des röhrenförmigen Durchlasses (46) ausdehnt, wobei das Ausdehnen des proximalen Abschnitts der Rahmenkonstruktion eine Drehung der distalen Verankerungshaken (14B) in den Herzmuskel bewirkt.

9. Vorrichtung nach Anspruch 5, wobei mindestens einige der Verankerungshaken (14B, 14C) proximale Verankerungshaken (14C) an einem proximalen Abschnitt der Rahmenkonstruktion (12) sind, wobei sich der proximale Abschnitt der Rahmenkonstruktion nach dem Verlassen des röhrenförmigen Durchlasses (46) ausdehnt, wobei das Ausdehnen des proximalen Abschnitts der Rahmenkonstruktion die Bewegung der proximalen Verankerungshaken weg von einer Mittellinie des röhrenförmigen Körpers bewirkt, so dass sich die proximalen Verankerungshaken in den Kontakt mit dem Herzmuskel bewegen.

10. Vorrichtung nach einem der vorhergehenden Ansprüche, wobei die Rahmenkonstruktion (12) einen Hauptrahmen (18) beinhaltet, der eine vertikale Achse (24) umgibt und eine Folge von Abschnitten (22, 22A, 22B) umfasst, die sich in Auf- und Abwärtsrichtung abwechseln, wobei das Ausdehnen der Rahmenkonstruktion die Abschnitte voneinander weg bewegt.

11. Vorrichtung nach Anspruch 10, wobei die Rahmenkonstruktion (12) von oben betrachtet zu verschiedenen nicht kreisrunden Formen verformbar ist, um ihre Positionierung in Herzkammerhohlräumen mit abweichenden nicht kreisrunden Formen zu ermöglichen.

12. Vorrichtung nach einem der Ansprüche 1 bis 9, wobei die Rahmenkonstruktion (12) von oben betrachtet eine vertikale Achse (24) umgibt und zu verschiedenen nicht kreisrunden Formen verformbar ist, um ihre Positionierung in Herzkammerhohlräumen mit abweichenden nicht kreisrunden Formen zu ermöglichen.

13. Vorrichtung nach einem der vorhergehenden Ansprüche, wobei die Rahmenkonstruktion (12) ferner einen Hauptrahmen (18) beinhaltet, der dafür gestaltet ist, sich weg von einer Mittellinie des röhrenförmigen Durchlasses auszudehnen, und wobei der Stützrahmen (20), wenn sich der Hauptrahmen im expandierten Zustand befindet, in einer durch den Hauptrahmen definierten Öffnung an selbigen montiert ist.

14. Vorrichtung nach Anspruch 13, wobei der Stützrahmen (20) in eine längliche Anordnung zusammengeklappt ist, wenn sich der Hauptrahmen (18) in zusammengeklapptem Zustand befindet.

15. Vorrichtung nach Anspruch 14, wobei der Stützrahmen mindestens zwei Elemente (32) aufweist, die im Verhältnis zueinander in scherenartiger Weise schwenken.

16. Vorrichtung nach einem der vorhergehenden Ansprüche, wobei sich die Membran (16) innerhalb der Rahmenkonstruktion (12) befindet, wenn sich die Rahmenkonstruktion im zusammengeklappten Zustand befindet.

17. Vorrichtung nach einem der vorhergehenden Ansprüche, wobei die Membran (16) einen Durchmesser von mindestens 3 cm aufweist.

## Revendications

1. Dispositif (10) d'amélioration de la fonction cardiaque, comprenant :
une structure cadre (12) comprenant un cadre de support (20), ladite structure cadre pouvant être déplacée d'un état replié, pour pouvoir introduire la structure cadre à travers un passage tubulaire (46) dans un coeur, à un état déployé, où la structure cadre présente une dimension transversale pour se rapprocher d'une dimension transversale d'une cavité ventriculaire ;
au moins un élément d'ancrage (14) raccordé à la structure cadre, l'élément d'ancrage ayant au moins une partie d'ancrage qui est positionnée et capable de s'ancrer à un tissu d'un myocarde du coeur, et ainsi d'ancrer au myocarde la structure cadre dans une position installée ; et
une membrane (16) qui peut être déplacée d'une condition pliée, pour pouvoir l'introduire à travers le passage tubulaire (46) dans le coeur, à une condition dépliée où elle présente une surface sensiblement plus grande qu'une surface transversale du passage tubulaire, ladite membrane étant fixée à la structure cadre dans une position lui permettant de former sensiblement une séparation entre les volumes de la cavité ventriculaire sur les côtés opposés de la membrane lorsque la structure cadre est dans la position installée,
où le cadre de support est suffisamment solide pour supporter la membrane et l'empêcher de se replier en cas de pressions systoliques maximales d'au moins 60 mmHg.

2. Dispositif selon la revendication 1, comprenant au moins deux éléments d'ancrage (14) raccordés à la structure cadre (12), chaque élément d'ancrage ayant au moins une partie d'ancrage qui est positionnée et capable de s'ancrer au tissu d'un myocarde du coeur, et ainsi d'ancrer au myocarde la structure cadre dans la position installée, les parties d'ancrage étant espacées les unes des autres pour permettre le positionnement de la structure cadre à un angle choisi par rapport à la cavité ventriculaire.

3. Dispositif selon la revendication 2, comprenant un troisième élément d'ancrage (14), les parties d'ancrage des éléments d'ancrage étant positionnées dans les angles d'un triangle pour permettre le positionnement de la structure cadre (12) avec la membrane (16) dans un plan choisi par rapport à la cavité ventriculaire.

4. Dispositif selon la revendication 2 à 3, où les parties d'ancrage ont des bouts pointus (26, 28) permettant de pénétrer dans le myocarde.

5. Dispositif selon la revendication 4, où au moins certains des éléments d'ancrage sont des crochets d'ancrage (14B, 14C).

6. Dispositif selon la revendication 5, où au moins certains des crochets d'ancrage (14B, 14C) sont des crochets d'ancrage distaux (14B) sur une partie distale de la structure cadre (12) qui tournent dans le myocarde pendant qu'un cathéter (44) formant le passage tubulaire (46) est retiré de la structure cadre.

7. Dispositif selon la revendication 6, où le retrait partiel du cathéter (44) d'une partie distale de la structure cadre (12) entraîne un déploiement de la partie distale de la structure cadre et un éloignement des crochets d'ancrage distaux (14B) par rapport à une ligne centrale du passage tubulaire (46), de sorte que les bouts pointus (26) des crochets d'ancrage distaux viennent en contact avec le myocarde, et un nouveau retrait du cathéter hors de la structure cadre entraîne une rotation des crochets d'ancrage distaux dans le myocarde.

8. Dispositif selon la revendication 7, où une partie proximale de la structure cadre (12) se déploie après être sortie du passage tubulaire (46), le déploiement de la partie proximale de la structure cadre entraînant une rotation des crochets d'ancrage distaux (14B) dans le myocarde.

9. Dispositif selon la revendication 5, où au moins certains des crochets d'ancrage (14B, 14C) sont des crochets d'ancrage proximaux (14C) sur une partie proximale de la structure cadre (12), la partie proximale de la structure cadre se déployant après être sortie du passage tubulaire (46), le déploiement de la partie proximale de la structure cadre entraînant un éloignement des crochets d'ancrage proximaux par rapport à une ligne centrale du passage tubulaire de sorte que les crochets d'ancrage proximaux viennent en contact avec le myocarde.

10. Dispositif selon l'une quelconque des revendications précédentes, où la structure cadre (12) inclut un cadre principal (18) entourant un axe vertical (24) et comprenant une séquence de sections (22, 22A, 22B) alternant dans des directions ascendantes et descendantes, le déploiement de la structure cadre éloignant les sections les unes des autres.

11. Dispositif selon la revendication 10, où, vue de dessus, la structure cadre (12) est déformable en diverses formes non circulaires pour permettre son positionnement dans des cavités ventriculaires ayant différentes formes non circulaires.

12. Dispositif selon l'une quelconque des revendications 1 à 9, où, vue de dessus, la structure cadre (12) entoure un axe vertical (24) et est déformable en diverses formes non circulaires pour permettre son positionnement dans des cavités ventriculaires ayant différentes formes non circulaires.

13. Dispositif selon l'une quelconque des revendications précédentes, où la structure cadre (12) inclut en outre un cadre principal (18) qui est configuré pour se déployer par rapport à une ligne centrale du passage tubulaire, et où lorsque le cadre principal est dans l'état déployé, le cadre de support (20) est monté sur le cadre principal dans une ouverture définie par le cadre principal.

14. Dispositif selon la revendication 13, où le cadre de support (20), lorsque le cadre principal (18) est dans l'état replié, est replié selon un agencement allongé.

15. Dispositif selon la revendication 14, où le cadre de support comporte au moins deux éléments (32) qui pivotent l'un par rapport à l'autre à la manière de ciseaux.

16. Dispositif selon l'une quelconque des revendications précédentes, où la membrane (16) est dans la structure cadre (12) lorsque la structure cadre est dans l'état replié.

17. Dispositif selon l'une quelconque des revendications précédentes, où la membrane (16) présente un diamètre d'au moins 3 cm.
